(19) Europäisches Patentamt
European Patent Office
Office européen des brevets

(11) Publication number: **0 329 194 B1**

(12) **EUROPEAN PATENT SPECIFICATION**

(45) Date of publication of patent specification: **24.03.93**

(51) Int. Cl.[5]: **C01F 7/00**, C07F 1/00, C07C 53/126, C08K 5/09, C08K 3/00, C10M 113/08, C10M 117/02

(21) Application number: **89104779.7**

(22) Date of filing: **10.06.85**

(60) Publication number of the earlier application in accordance with Art.76 EPC: **0 169 357**

(54) **Metallo-organo aluminates as additives.**

(30) Priority: **11.06.84 US 619442**
**11.06.84 US 619427**

(43) Date of publication of application:
**23.08.89 Bulletin 89/34**

(45) Publication of the grant of the patent:
**24.03.93 Bulletin 93/12**

(84) Designated Contracting States:
**BE CH DE FR GB IT LI NL SE**

(56) References cited:
**EP-A- 0 103 034**
**EP-A- 0 142 773**
**GB-A- 1 190 907**
**US-A- 4 348 297**

(73) Proprietor: **THE DOW CHEMICAL COMPANY**
**2030 Dow Center Abbott Road P.O. Box 1967**
**Midland Michigan 48640-1967(US)**

(72) Inventor: **Kar, Kishore K.**
**4512 North Saginaw Road**
**Midland Michigan 48460(US)**
Inventor: **Burba, John L. III**
**168 Dallas**
**Angleton Texas 77515(US)**
Inventor: **Bauman, William C.**
**2900 Braley Court**
**Midland Michigan 48640(US)**

(74) Representative: **Casalonga, Axel et al**
**BUREAU D.A. CASALONGA - JOSSE Morassistrasse 8**
**W-8000 München 5 (DE)**

EP 0 329 194 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention concerns crystalline lithium aluminate compositions useful as additives for organic materials.

Crystalline aluminate compositions conforming generally to the empirical formula $Li^{+}(RCOO^{-})\cdot 2Al(OH)_3\cdot nH_2O$, where $RCOO^{-}$ represents an organic acid anion, and $nH_2O$ represents any waters of hydration, are disclosed, inter alia, in U.S. 4,348,295, U.S. 4,348,296, and U.S. 4,348,297.

The following patents are believed to be representative of some of the prior art regarding extreme pressure lubricant additives or lithium aluminate compounds: U.S. 2,621,159; U.S. 3,001,939; U.S. 3,093,584; U.S. 3,318,808; U.S. 3,565,802; U.S. 3,909,426; U.S. 3,984,599; U.S. 3,997,454; U.S. 4,116,856; U.S. 4,116,858; U.S. 4,159,311; U.S. 4,221,767; U.S. 4,293,430; U.S. 4,347,327; U.S. 4,321,065; U.S. 4,376,100; and U.S. 4,381,349.

Also disclosed are crystalline $LiX\cdot 2Al(OH)_3\cdot nH_2O$ compounds and derivatives thereof, e.g., where the X anion represents OH, halide, halo acid, inorganic acid, organic acid and others. The compounds are referred to generally as "lithium aluminates" and are prepared, principally, by reacting lithium salts with hydrous alumina and forming crystalline $LiX\cdot 2Al(OH)_3\cdot nH_2O$ which in some cases are of the "two-layer" variety and in some cases of the "three-layer"variety, depending on the particular method or materials employed. Methods for preparing these known crystalline lithium aluminates, of the $LiX\cdot 2Al(OH)_3\cdot nH_2O$ formula, both 2-layer and 3-layer varieties, and anion exchanges or replacements in the crystals, are disclosed in the patents identified above.

In this present disclosure the expression "lithium stearate aluminate" (also "LSA") is a crystalline compound of the formula, $Li^{+}(RCOO^{-})\cdot 2Al(OH)_3\cdot nH_2O$, where $RCOO^{-}$ is the negative-valent carboxylate radical of stearic acid. It is prepared in accordance with the procedure disclosed in U.S. 4,348,295 or U.S. 4,348,297 where crystalline $LiOH\cdot 2Al(OH)_3\cdot nH_2O$, material is reacted with stearic acid, thereby replacing the $OH^{-}$ (attached to the Li) with $RCOO^{-}$.

Lithium stearate aluminates (LSA) are organic-inorganic (60:40) hybrid crystalline materials conforming substantially to the empirical formula $LiX\cdot 2Al(OH)_3\cdot nH_2O$, where X is an anion (stearate) and $nH_2O$ represents water of hydration. These are 2- or 3-layer unit cell structures. The particle size is usually from 150 Å to 5000 Å. TGA studies have shown that it decomposes at 300°C. X-ray defraction and SEM analyses have revealed its platelet structure. These LSA compounds also include the compound of formula I and II as defined below.

The present invention relates to the use of a crystalline lithium aluminate as an additive to an organic material by uniformly dispersing in said organic material (not being a lubrication fluid), as small particles, at least one crystalline aluminate conforming substantially to the formula:

I. $(LiA_x)_y\cdot 2Al(OH)_3\cdot nH_2O$

wherein A is one or more anions and/or negative-valence radicals or mixtures thereof;

wherein, in formula I, x is a quantity of A ions or radicals sufficient to substantially satisfy the valence requirements of Li;

wherein n is zero or the number or waters of hydration;

wherein y is a numerical value at least sufficient to maintain the crystalline structure.

The preferred composition of the present invention comprises a substantially uniform mixture of an organic material and at least one compound of formula I as defined above.

An especially preferred composition comprises a mixture of an organic material and a crystalline lithium aluminate of the formula

II. $$(Li(OH)_{1-gp}E_p^{-g})_y\cdot 2Al(OH)_3\cdot nH_2O$$

wherein E is at least one anion or negative-valence radical other than $OH^{-}$, with a valence of -g;

wherein p is greater than or equal to zero and is less than or equal to unity; and

wherein y and n are defined as above.

The aluminate crystal of formula I or II may be of the two-layer or three-layer variety or may be a mixture of the two varieties.

As shown in the above patents, hydrous alumina, represented by the formula $Al(OH)_3$, may be suspended in an ion exchange resin and then reacted with aqueous LiOH at elevated temperature to form crystalline $LiOH \cdot 2Al(OH)_3$. It is understood, of course, that the so-formed crystalline aluminates, being in contact with water, have waters of hydration attached.

The above patents also disclose that the crystalline $LiOH \cdot 2Al(OH)_3$ is beneficially converted to $LiX \cdot 2Al(OH)_3$, where X is a halogen, i.e. Cl, Br, or I.

It is also disclosed that the crystalline $LiOH \cdot 2Al(OH)_3$, whether supported within or on a substrate, or prepared in the absence of a substrate, is beneficially converted to other lithium aluminates by reactions which replace the OH radicals with other anions or radicals.

Substrates, in addition to ion exchange resins, include, e.g., inorganic substrates (which are substantially inert to the reactions involved in preparing the $(LiA_x)_y \cdot 2Al(OH)_3 \cdot nH_2O$), inert organic or inert polymeric substrates, and inert metallic substrates.

The "neat" prepared subject compounds, i.e. in the absence of a substrate, are also useful in the present invention and usually allow larger aggregates or stacks of the crystals in the crystalline structure.

The anions contemplated by A in the above formula (including halide and hydroxyl) include the anions of soluble inorganic acids, mineral acids, organic acids, or anions of the salts of such acids.

The anions of inorganic acids and mineral acids include, for example, $SO_4^{--}$ , $HCO_3^-$ , $BO_2^-$ , $H_2PO_4^-$ , $HPO_4^{--}$ , $ClO_4^-$ , $HCrO_4^-$ , $NO_3^-$ , $SO_3^{--}$ , $HSO_3^-$ , $NO_2^-$ , $H_2AsO_4^-$ ,. $HAsO_4^{--}$ , $F^-$, $HS^-$, $ClO_3^-$ , $H_2PO_3^-$ , $HPO_3^{--}$ $^-$, $H_3P_2O_7^-$ , $MnO_4$, $H_2P_2O_7^{--}$ $^-$, $HP_2O_7^{--}$ $^-$, $NH_2SO_3^-$ , $H_2PO_4^-$ , $PO_4^{--}$ $^-$, and the like.

The anions of organic acids may be derived, for example, from monobasic acids (RCOOH), dibasic acids (HOOC-COOH or HOOC-R-COOH), tribasic acids (HOOC-R(COOH)-COOH) where R is a substituted or unsubstituted hydrocarbon moiety, and other multibasic organic acids, such as ethylenediamine tetraacetic acid, acrylic acid polymers and copolymers, pyromellitic acid, and the like. Examples of monobasic acids are, for instance, formic acid, acetic acid, chloroacetic acid, dichloroacetic acid,trichloroacetic acid, acrylic acid, methacrylic acid, crotonic acid, butyric acid, propionic acid, tartaric acid, hexanioc acid, fatty acids (such as stearic acid), aromatic and cyclic acids (such as benzoic, toluic, salicylic, gallic, cinnamic acid), and the like. Examples of dibasic acids are, for instance, oxalic acid, malonic acid, fumaric acid, malic acid, maleic acid, succinic acid, terephthalic acid, pimelic acid, and the like. Citric acid is an example of a tribasic acid, $HOOCCH_2C(OH)(COOH)CH_2COOH$. Quadribasic acids, such as pyromellitic acid, are also contemplated by the present invention. Hydroxy carboxylic acids, such as glycollic acid, lactic acid, tartaric acid, and malic acid are also contemplated by the present invention. Organic radicals with inorganic substituents, such as $CH_3SO_3^-$ , $CH_3PO_3^{--}$ , and $C_6H_{11}SO_3^-$ are also contemplated by the present invention.

Crystalline lithium aluminates conforming to the formula $Li(RCO\overline{O})_y \cdot 2Al(OH)_3 \cdot nH_2O$, wherein $RCO\overline{O}$ is a fatty acid anion, y is the number of Li atoms for each 2 Al atoms and n is zero or a positive amount of waters of hydration, are found to be useful as additives to organic fluids as thickeners, as viscosity control agents, as compatabilizers, and/or as dispersing agents. These aluminates are especially useful as additives to silicone oils, synthetic oils, organics, and hydrocarbons, most especially aliphalic hydrocarbons such as mineral oils, petroleum oils, motor oils, diesel oils, vegetable oils, and the like. In those aluminates wherein the fatty acid anion is derived from fatty acids having 10 or more carbon atoms in the aliphatic carbon chain, or branched-chains, the aluminate is, itself, a useful grease or lubricant.

As used within the purview of this disclosure, the $Li(RCOO)_y \cdot 2Al(OH)_3 \cdot nH_2O$ compounds include the 2-layer and 3-layer varieties such as disclosed in U.S. 4,348,295 and U.S. 4,348,296. When written as $Li(RCOO)_y \cdot 2Al(OH)_3 \cdot nH_2O$, the subscript y is used (as in U.S. 4,348,297) to indicate the number of Li atoms per each 2 Al atoms; the value of y is preferably 1.0, but may be from 0.5 to 2, depending on how the crystals are prepared and on how much (if any) Li values have been leached or exchanged out of the crystals. In some cases the value of n can be virtually zero, indicating that waters of hydration are essentially absent, but in the absence of an intensive drying procedure, the value of n is usually in the range of 0 to 6.

The $RCOO^-$ anion in the lithium aluminate crystal may be any fatty acid wherein R is an aliphatic carbon chain or branched-chain, having one or more carbon atoms. In those instances wherein it is desired that the aluminate compound be employed, e.g. as a thickener, a gelling agent, a processing aid, a dispersing agent, and the like, in various oils, water dispersions, organic fluids, or as a grease or lubricant itself, it is preferred that the $RCOO^-$ anion contain more than 8 carbon atoms, more preferably 12 or more carbon atoms, most preferably 14 to 22 carbon atoms.

The lithium aluminates of the present invention can also be added to polymers, waxes and paraffins which can be sufficiently fluidized at a temperature, generally, less than about 250-300 °C to permit adequate mixing with the aluminate. These mixtures are useful, e.g., as lubricants, mold release agents, fire-retarding additives, and polymer additives. These lithium aluminates also serve to reinforce polymers or resins or other solidified materials to which they are added.

Of the fatty acids which are the source of the $RCOO^-$ anions of the crystalline lithium aluminates of the present invention, those which have from 1 to 8 carbon atoms in their molecule are at least partially soluble in water at 20°C, but those with 9 or more carbon atoms in their molecule are practically insoluble in water at 20°C. Thus, in preparing $Li(RCOO^-) \cdot 2Al(OH)_3 \cdot nH_2O$ by the reaction of $CH_3(CH_2)_xCOOH$ (x is at least 7) with $LiOH \cdot 2Al(OH)_3 \cdot nH_2O$, a solvent or reaction medium other than water may be used. A convenient solvent or carrier is alcohol, such as isopropanol, though other solvents or carriers for the fatty acid may be used, such as hexane, toluene, oils (e.g. mineral oils), ethers, halocarbons, silicone fluids, and the like. The fatty acid itself, so long as it is at a temperature at which it is molten, can serve as its own reaction medium. For example, nonylic acid melts at about 12.5°C and stearic acid melts at about 69°C.

A modicum of success is achieved by carrying out the long chain $RCOO^-$ intercalations in a water carrier if the fatty acid (molten or solid) is finely dispersed in the water, or a solution of the fatty acid is finely dispersed in water, and conducting the reaction with the crystalline lithium aluminate, preferably with stirring and at elevated temperature.

Of particular interest are $Li(RCOO^-)_y \cdot 2Al(OH)_3 \cdot nH_2O$ crystals wherein the $RCOO^-$ radical is oleic acid anion, stearic acid anion, linoleic acid anion, linolenic acid anion, benzioc acid anion, and the like. These aluminates are thin platey structures which are substantially thermally stable to temperatures of 300°C-400°C. Some of them have about the same consistency as candle wax or soap and are useful as lubricants or greases at moderately high temperatures where many known hydrocarbon greases may lose their viscosity to the extent of being virtually ineffective as lubricants. Furthermore, these aluminates exhibit an ability to beneficially thicken hydrocarbon oils. For instance, 3-layer lithium oleate aluminate and 3-layer lithium stearate aluminate are successfully dispersed in mineral oil, motor oil, and diesel oil by adding about 30 g. of the material to 300 g. of the oil by the action of an ultrasonic disperser operated about 12 minutes at a temperature of 100°C. Stable water-in-oil dispersions can be prepared by dispersing lithium stearate aluminate in oils, e.g. diesel oil, motor oil, mineral oil, hydraulic fluids, and the like, which contain, e.g. 10 percent by volume $H_2O$.

The crystalline lithium aluminates of the present invention are prepared according to the following general procedure.

Crystalline or amorphous hydrous alumina, denoted as $Al(OH)_3$, is reacted at elevated temperature to form crystalline $LiOH \cdot 2Al(OH)_3 \cdot nH_2O$ in an aqueous medium. The beginning hydrous alumina may be unsupported by a substrate, or may be supported on a substrate, or may be dispersed or suspended within a porous substrate. The reaction between the hydrous alumina and the LiOH may take place at room temperature but to assure that the reaction is substantially completed within a reasonable length of time, an elevated temperature of at least 50°C, preferably at least 75°C, should be used. The amount of LiOH should not be in such excess that the aluminate is caused to precipitate outside the pores. The aqueous media may contain other ingredients and, if they are substantially inert or do not interfere with the desired reaction, are permissible. Insoluble, substantially inert particles may be present in the aqueous medium and may serve as a substrate for the $LiOH \cdot 2Al(OH)_3$ as it is formed. Choice of a substrate (if used) is dependent, of course, on the intended use of, or application of, the crystalline $LiOH \cdot 2Al(OH)_3 \cdot nH_2O$.

The present invention is not limited to a particular means for providing the beginning hydrous alumina for reaction with the LiOH. For example, the pores of a substrate may be substantially filled with $Al(OH)_3$ by growing seeds of $Al(OH)_3$ in the pores from an aqueous solution of sodium aluminate.

The crystalline $LiOH \cdot 2Al(OH)_3 \cdot nH_2O$ is then reacted in aqueous medium with anions or negative-valence radicals (A as hereinbefore described) having a valence of 1, 2, or 3 or more to form the $(LiA_x)_y \cdot 2Al(OH)_3 \cdot nH_2O$ compounds of the present invention. A monovalent anion or radical yields $(LiA)_y \cdot 2Al(OH)_3 \cdot nH_2O$. A divalent anion or radical yields $(LiA_{\frac{1}{2}})_y \cdot 2Al(OH)_3 \cdot nH_2O$. A trivalent anion or radical yields $(LiA_{1/3})_y \cdot 2Al(OH)_3 \cdot nH_2O$. Radicals of valence greater than 3 are similarly stoichiometrically balanced. The value of y is normally 1, but the actual value of y may vary over the range of 0.5 to 2.0, especially 0.5 to 1.2.

The novel compositions of the present invention are prepared by substantially uniform mixing of an organic material not being a lubrication fluid and at least one crystalline lithium aluminate compound of the formula

I. $(LiA_x)_y \cdot 2Al(OH)_3 \cdot nH_2O$

wherein A, x, y and n are as hereinbefore defined.

The organic material contemplated in the composition of the present invention includes a hydrocarbon, a polymer, a resin, a silicone, a thermoplastic material, a thermosetting material and the like.

The hydrocarbon contemplated by the organic material of the present invention includes aliphatic, paraffinic, bicyclic, alicyclic, aromatic, alkane, alkene, arylene, isoalkylene, isoalkane, or isoalkene hydrocarbons, including those which are substituted or unsubstituted, and including those which contain heteroatoms of the group consisting of N, S, O, Si, P, F, Cl, Br, and I.

The thermoplastic material contemplated as the organic material in the present invention includes a hydrocarbon wax, a paraffin, an olefin polymer, an olefin copolymer, a vinyl polymer, a vinyl copolymer, a polycarbonate, a polyalkyleneimine, a polyether, an epoxy, a polyurethane, a polysulfone, a polysiloxane, a polyterpene, a polyfluorocarbon, a polyimide, a silicone resin, a polyamide, a polyalkyleneoxide, or a polyacrylate.

The thermosetting material contemplated as the organic material in the present invention includes an epoxy, an epoxy-novolac, a vinylester, a polyester, a polyurethane, a polyether, a glyptal resin, a phenolic resin, a ureaformaldehyde resin, or a urea condensation resin.

The organic material may be used as such or dissolved in a suitable solvent. The organic material may itself be a liquid at ambient temperatures and pressure and may contain at least a trace amount of halogen present therein.

The mixing of the organic material and the crystalline lithium aluminate compound can be done by any known mixing means such as by the use of an agitator, a recycle pump, a sonic mixer, an in-line mixer and the like.

The embodiments of tests and examples which follow are only to illustrate the invention.

## Example 1

An aqueous solution of $AlCl_3$ was reacted with $NH_4OH$ thereby precipitating $Al(OH)_3$. The $Al(OH)_3$ was washed with $H_2O$ to wash out $NH_4Cl$ and a slurry of the $Al(OH)_3$ in water was reacted with LiOH at elevated temperature (95 °C) to form crystalline $LiOH{\bullet}2Al(OH)_3{\bullet}nH_2O$.

A portion of the $LiOH{\bullet}2Al(OH)_3{\bullet}nH_2O$ slurried in water was titrated to pH = 6 with $CCl_3COOH$ (trichloroacetic acid) to form crystalline $Li(CCl_3COO){\bullet}2Al(OH)_3{\bullet}nH_2O$.

In a similar manner other lithium aluminates are prepared wherein the anion is $BO_2^-$, $NO_3^-$, $HCO_3^-$, $H_2PO_4^-$, $SO_4^{--}$, $F^-$, $CH_2ClCOO^-$, $CCl_2HCOO^-$, and the like.

X-ray diffraction patterns on the above products, and other products disclosed herein, indicate a crystalline material falling into the hexagonal crystal system with an interlayer distance of at least 7.5Å. This distance is dependent on the size of the anion. These are 2- or 3-layer unit cell structures. The particle diameter is usually from 150Å to 10000Å. X-ray diffraction and scanning electron microscopic analysis have revealed its platelet structure. The ratio of the length to the thickness of these platelets can be between 1 and 1500. White powders or particles are generally produced, but tinted or colored products are not precluded from this invention.

The number of waters of hydration in the crystalline aluminates used in the present invention is generally within the range of 0 to 6.

## Example 2

In one particular embodiment of the present invention, 73 g. of crystalline 3-layer $LiOH{\bullet}2Al(OH)_3{\bullet}nH_2O$ (wherein n is about 3) was dispersed in 400 ml. of drum grade 2-propanol and 114 g. of commercially available stearic acid (95 percent purity) was added. The mixture was stirred at 40 °C for 1 hour, then filtered and the product analyzed. By analysis it is found that lithium stearate aluminate was formed, conforming to the formula $Li(RCOO){\bullet}2Al(OH)_3{\bullet}nH_2O$ (wherein n is 0), the product also contained a small amount of unreacted stearic acid which can be substantially removed by washing with 2-propanol.

## Example 3

The procedure of Example 2 was repeated using crystalline 2-layer $LiOH{\bullet}2Al(OH)_3{\bullet}nH_2O$ and substantially the same results were obtained except for the difference in the number of crystal layers.

## Example 4

The procedure of Example 2 was repeated except that the amount of stearic acid used was less than enough to replace all the OH anions in the $LiOH{\bullet}2Al(OH)_3{\bullet}nH_2O$ crystal, and the product made conforms to the formula $Li(OH)_{\frac{1}{2}}(RCOO)_{\frac{1}{2}}{\bullet}2Al(OH)_3{\bullet}nH_2O$. This compound finds utility as an additive to organics and

hydrocarbons, e.g., as a thickener, an acid ion scavenger, a viscosity-adjusting agent, a lubrication agent, as emulsion stabilizers, as solids dispersing agents, and the like.

Example 5

100 ml of a mineral oil having a viscosity of about 50 centipoise was mixed with lithium aluminium hydroxy stearate at a concentration of about 5% weight. The mixture was sheared in a high-shear mixer for about 2 hours. The resulting material was a grease that would not drip at 300° C.

Example 6

A 5g sample of lithium aluminium hydroxy methacrylate (LAHM) was dispersed in 100g of a vinyl ester resin using a paint mill. The resin was then cast into a sheet and cured. The tensile strength of the resulting LAHM/resin mixture was 20% greater than that of the polyester resin without LAHM.

**Claims**

**1.** Use of a crystalline lithium aluminate as an additive to an organic material comprising:
uniformly dispersing in said organic material not being a lubrication fluid, as small particles, at least one crystalline aluminate conforming substantially to the formula

I. $(LiA_x)_y \cdot 2Al(OH)_3 \cdot nH_2O$

wherein A is one or more anions and/or negative-valence radicals or mixtures thereof;
wherein, in formula I, x is a quantity of A ions or radicals sufficient to substantially satisfy the valence requirements of Li;
wherein n is zero or the number of waters of hydration;
wherein y is a numerical value at least sufficient to maintain the crystalline structure.

**2.** The use of the compound according to claim 1 wherein A = $RCOO^-$ and contains more than 8 carbon atoms.

**3.** The use of the compound according to claim 2 wherein $RCOO^-$ contains 14-22 carbon atoms.

**4.** The use of the compound according to any one of claims 1-3 wherein the compound is used in an amount of 5-10% by weight.

**5.** The use of the compound according to any of claims 1-4 wherein the organic material is a hydrocarbon or a macromolecular compound.

**6.** A composition which comprises a substantially uniform mixture of an organic material not being a lubrication fluid and at least one compound of formula I as defined in any of claims 1-4.

**7.** The composition of Claim 6 which comprises a mixture of an organic material and a crystalline lithium aluminate of the formula

$$II. \quad (Li(OH)_{1-gp}E_p^{-g})y \cdot 2Al(OH)_3 \cdot nH_2O$$

wherein E is at least one anion or negative-valence radical other than $OH^-$, with a valence of -g;
wherein p is greater than or equal to zero, and is less than or equal to unity; and
wherein y and n are defined as in Claim 1.

**8.** The composition according to claim 6 or 7 wherein the organic material is a hydrocarbon or a macromolecular compound.

## Patentansprüche

**1.** Verwendung eines kristallinen Lithiumaluminates als Zusatzstoff zu einem organischen Material, umfassend:
gleichförmiges Dispergieren in diesem organischen Material, das kein Schmierfluid ist, in Form von kleinen Teilchen wenigstens eines kristallinen Aluminates entsprechend im wesentlichen der Formel:

I. $(LiA_x)_y \cdot 2Al(OH)_3 \cdot nH_2O$

worin A ein oder mehrere Anionen und/oder negativwertige Reste oder Mischungen hiervon bedeuten;
worin, in der Formel I, x eine ausreichende Menge von A-Ionen oder -Resten, um im wesentlichen den Wertigkeitserfordernissen von Li zu genügen, bedeutet;
worin n null oder die Anzahl der Hydrationswasser bedeutet;
worin y ein numerischer Wert ist, der wenigstens ausreicht, um die kristalline Struktur beizubehalten.

**2.** Verwendung der Verbindung nach Anspruch 1, worin A = $RCOO^-$ und mehr als 8 Kohlenstoffatome enthält.

**3.** Verwendung der Verbindung nach Anspruch 2, worin $RCOO^-$ 14-22 Kohlenstoffatome enthält.

**4.** Verwendung der Verbindung nach einem der Ansprüche 1-3, worin die Verbindung in einer Menge von 5-10 Gew. -% verwendet wird.

**5.** Verwendung der Verbindung nach einem der Ansprüche 1-4, worin das organische Material ein Kohlenwasserstoff oder eine makromolekulare Verbindung ist.

**6.** Zusammensetzung, welche eine im wesentliche gleichförmige Mischung von einem organischen Materials, das kein Schmierfluid ist, und von wenigstens einer Verbindung der Formel I, wie in einem der Ansprüche 1-4 definiert, umfaßt.

**7.** Zusammensetzung nach Anspruch 6, welche eine Mischung eines organischen Materials und eines kristallinen Lithiumaluminates der Formel umfaßt:

II. $(Li(OH)_{1-gp}E_p^{-g})_y \cdot 2Al(OH)_3 \cdot nH_2O$

worin E wenigstens ein von $OH^-$ verschiedenes Anion oder negativwertiger Rest mit einer Wertigkeit von -g ist;
worin p größer oder gleich 0 ist und weniger als oder gleich eins ist; und
worin y und n die in Anspruch 1 angegebene Bedeutung haben.

**8.** Zusammensetzung nach Anspruch 6 oder 7, worin das organische Material ein Kohlenwasserstoff oder eine makromolekulare Verbindung ist.

## Revendications

**1.** Utilisation d'un aluminate de lithium cristallin comme adjuvant d'un matériau organique, par dispersion uniforme dans ce matériau organique (qui n'est pas un fluide lubrifiant), sous forme de petites particules, d'au moins un aluminate cristallin correspondant pratiquement à la formule :

I $(LiA_x)_y \cdot 2Al(OH)_3 \cdot nH_2O$

dans laquelle A représente un ou plusieurs anions et/ou radicaux à valence négative ou leurs mélanges,
dans laquelle formule I x représente une quantité d'ions ou de radicaux A suffisante pour équilibrer pratiquement la valence de Li,
dans laquelle n vaut zéro ou représente le nombre de molécules d'eau d'hydratation, et

dans laquelle y représente un nombre au moins suffisant pour que soit maintenue la structure cristalline.

2. Utilisation du composé selon la revendication 1, dans lequel A représente $RCOO^-$ et comporte plus de 8 atomes de carbone.

3. Utilisation du composé selon la revendication 2, dans lequel $RCOO^-$ comporte de 14 à 22 atomes de carbone.

4. Utilisation du composé selon l'une quelconque des revendications 1 à 3, ce composé étant utilisé en une quantité de 5 à 10 % en poids.

5. Utilisation du composé selon l'une quelconque des revendications 1 à 4, le matériau organique étant un hydrocarbure ou un composé macro-moléculaire.

6. Composition qui comporte un mélange pratiquement uniforme d'un matériau organique, qui n'est pas un fluide lubrifiant, et d'au moins un composé de formule I défini dans l'une quelconque des revendications 1 à 4.

7. Composition selon la revendication 6, qui comprend un mélange d'un matériau organique et d'un aluminate de lithium cristallin de formule

II $(Li(OH)_{1-gp}E_p^{-g})_y \cdot 2Al(OH)_3 \cdot nH_2O$

dans laquelle E représente au moins un anion ou un radical à valence négative, autre que $OH^-$ et de valence -g,
dans laquelle p est supérieur ou égal à zéro, et inférieur ou égal à l'unité, et
dans laquelle y et n sont définis comme dans la revendication 1.

8. Composition selon la revendication 6 ou 7, dans laquelle le matériau organique est un hydrocarbure ou un composé macro-moléculaire.